# EUROPEAN PATENT SPECIFICATION

(11) **EP 0 294 114 B1**
(45) Date of publication and mention of the grant of the patent: **11.09.1996**
(21) Application number: 88304813.4
(22) Date of filing: 27.05.1988
(51) Int. Cl.: C07H 19/19, C07H 19/20, A61K 31/70

(54) **Antiviral compounds**
Antivirale Verbindungen
Composés antiviraux

(30) Priority: 30.05.1987 GB 8712745
(43) Date of publication of application: 07.12.1988
(73) Proprietor: THE WELLCOME FOUNDATION LIMITED, London NW1 2BP (GB)
(72) Inventor: Krenitsky, Thomas Anthony, Chapel HillNorth Carolina 27514 (US); Koszalka, George Walter, Chapel HillNorth Carolina 27514 (US); Jones, Lynda Addington, CaryNorth Carolina 27513 (US); Averett, Devron Randolph, RaleighNorth Carolina 27608 (US); Moorman, Allan Ray, DurhamNorth Carolina 27713 (US)
(74) Representative: Volckman, Janis Florence

(56) References cited:
- EP-A- 0 097 376
- EP-B- 0 002 192
- US-A- 4 495 180
- ANNALS OF THE NEW YORK ACADEMY OF SCIENCES, vol. 284, 1977; TH.H. HASKELL, pp. 81-90

## Description

The present invention relates to certain substituted purine arabinosides, and physiologically acceptable derivatives thereof in particular esters, and their use in the treatment of certain DNA virus diseases.

Varicella-herpes zoster virus (VZV) which causes chicken-pox and shingles, is a DNA virus of the herpes family. Varicella (chicken-pox) is the primary disease produced by VZV in a host without immunity; it is usually a mild illness of young children which is manifested as fever and an itching rash. Herpes zoster (shingles) is the recurrent form of the disease occurring in adults who were previously infected with the varicella-zoster virus. The clinical manifestations of this infection are characterised by neuralgia and a vesicular skin rash that is unilateral and dermatomal in distribution. Spread of inflammation may lead to paralysis or convulsions, and coma if the meninges become affected.

In immunodeficient patients the virus may disseminate causing serious often fatal illnesses. The cause of immunodeficiency may be drugs used on transplant patients or in the treatment of malignant neoplasms, or diseases, such as AIDS, which destroy the immune system, leaving the victim vulnerable to infections which would not otherwise be dangerous.

Cytomegalovirus (CMV) is another virus of the herpes family Infection may be acquired in infancy or early adulthood and, in the foetus, intra-uterine infection is probably the commonest form of infection, but up to 90% of congenital infections are asymptomatic at birth. Primary infection of the mother during pregnancy is generally considered to pose the greatest risk to the unborn child, whereas in reactivation the foetal infections are usually clinically silent. Clinical effects range from death and gross disease (microcephaly, hepatosplenomegaly, jaundice, mental retardation) through failure to thrive, susceptability to chest and ear infections to a lack of any obvious ill effect. In young adults infection may well go unnoticed or manifest as a glandular fever like disease resulting from close physical contact.

Equally serious infections may also occur due to reactivation of the dormant virus in immuno-compromised patients as described for VZV infections. Such infections result in increased morbidity and fatality from retinitis, pneumonitis and gastro-intestinal disorders.

It has now been found that certain purine-arabino nucleosides described below characterised by the presence of groups substituted at the 2- and 6-positions of the purine ring have potent activity against human virus infections particularly those caused by varicella zoster virus (VZV) or cytomegalovirus (CMV).

Certain substituted purine-arabino nucleosides, in particular 9-β-D-arabinofuranosyl-6-molhoxy-9H-purine, 9-β-D-arabinofuranosyl-6-pyrrolidino-9H-purine, 9-(β-D-arabinofuranosyl-6-methylamino-9H-purine and 9-β-D-arabinofuran- osyl-6-dimethylamino-9_H-purine described hereinafter for their use in the treatment of VZV and CMV infections, have previously been described in J.Org.Chem. Vol 27 3274-9 (1962); Cancer Treatment Rep. 60(10), 1567-84, (1976); Tetrahedron 40(4), 709-13, (1984); Canada J. Biochem. 43(1 1-15, (1965); J.Med.Chem. 12, 498-504, (1969); J. Biol. Chem. 251(13), 4055-61, (1976); Ann. N.Y. Acad. Sci. 284, 81-90, (1977); EP002192; US3666856; US4371613; US3758684.

Haskell (Annals. New York Acad. Sci. 1977, 284, 81) discloses the clinical efficacy of 9-p-D-arabinofuranosylad- enine analogs against VZV and CMV. This reference briefly discusses modification of this compound at the 6-positon to produce N-acylated analogs, Schiff base analogs, 6-hydroxy, 6-halo or 6-hydrogen analogs. There is, however, no reference to any substitution at the 2-position of the ring system and hence to the 9-p-D-arabinofuranosyl-2-amino-6-methoxy-9H-purine of the present invention, nor to the esters of the present invention. This reference concludes that a minimal amount of substitution in the pyrimidine ring of vidarabine is allowable if in vitro antiviral activity is to be maintained.

European Patent No. 0002192 relates to enzymatic synthesis of purine and purine derivative arabinonucleosides which may be used as anti-viral agents. While this citation refers to generic purine arabinosides and to a number of particular compounds which may be enzymatically produced, there is no specific reference to 9-p-D-arabinofuranosyl-2-amino-6-methoxy-9H-purine nor to any of the esters of the present invention.

Thus in the first aspect of the present invention there is provided the use of a compound of formula (I): 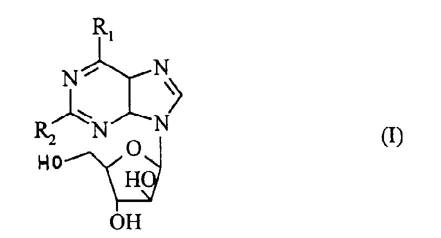 wherein R₁ is methoxy and R₂ is hydrogen or amino, and physiologically acceptable derivatives thereof in the manufacture of a medicament for the treatment or prophylaxis of human viral infections caused by VZV

In another aspect of the present invention there is provided 9-p-D-arabinofuranosyl-2-amino-6-methoxy-9H-purine or a physiologically acceptable derivative thereof.

In yet another aspect of the present invention there is provided a pharmaceutically acceptable ester of a compound of formula (I) selected from:
9-(5-O-(4-methylbenzoyl)-β-D-arabinofuranosyl)-6-methoxy-9H-purine
9-(5-O-(4-aminobenzoyl)-[β-D-arabinofuranosyl)-6-methoxy-9H-purine; or
6-methoxy-9-(2-O-pentanoyl-[β-D-arabinofuranosyl-9H-purine.

In a further aspect of the present invention there is provided 9-p-D-arabinofuranosyl-2-amino-6-methoxy-9H-purine for use in medicine.

The following compounds are therefore preferred compounds of the present invention by virtue of their potent antiviral activity against VZV:
1) 9-[β-D-Arabinofuranosyl-6-methoxy-9H-purine, and
2) 9-[3-D-Arabinofuranosyl-2-amino-6-methoxy-9H-purine.

In a further aspect of the present invention there are provided Pharmaceutically acceptable derivatives of the compounds of formula (I) include any pharmaceutically acceptable ether, salt, ester or salt of such ester, or any other compound which, upon administration to a human subject is capable of providing (directly or indirectly) a compound of formula (I) or an antivirally active metabolite or residue thereof.

The pharmaceutically acceptable esters of the above compounds of formula (I) are particularly preferred since they are capable of providing high levels of the parent compound in the plasma of a subject after oral administration. The present invention particularly provides as a preferred class of novel compounds the pharmaceutically acceptable esters of compound 1) above formed by esterification of the 2'-, 3'- and/or 5'- hydroxy group of the arabino-sugar moiety.

The following esters and ethers are preferred novel compounds of the present invention :
3. 9-(5-O-(4-Methylbenzoyl)-(β-D-arabinofuranosyl)-6-methoxy-9H-purine.
4. 9-[5-O-(4-Nitrobenzoyl)-[β-D-arabinofuranosyl]-6-methoxy-9H-purine.
5. 9-[5-0-(4-Aminobenzoyl)-(β-D-arabinofuranosyl]-6-methoxy-9H-purine.
6. 6-Methoxy-9-(2-O-pentanoyl-(β-D-arabinofuranosyl)-9H-purine.
7. 6-Methoxy-9-[3,5-O-(1,1,3,3-tetraisopropyl-1,3-disiloxan-1, 3-diyl) -[β-D-arabinofuranosyl]-9H-purine.

The purine nucleosides of formula (I) and their derivatives will be hereinafter referred to as compounds according to the invention or as active ingredients.

In a further, preferred aspect of the present invention, there is provided the use of a compound according to the invention in the manufacture of a medicament for the treatment or prophylaxis of human viral infections caused by VZV or CMV

The present invention further provides a method for the treatment or prophylaxis of VZV and CMV infections in a human subject which comprises administering to the said human subject an effective amount of a compound according to the invention.

The method hereinbefore described includes inhibiting the replication of VZV or CMV viruses in host cells of a mammal which comprises applying an effective virus replication inhibiting amount of the compound of formula (I), or a pharmaceutically acceptable derivative thereof, to the infected cells.

Examples of the clinical conditions caused by VZV and CMV infections which may be treated in accordance with the invention include those referred to above.

The compound of formula (I) and pharmaceutically acceptable derivatives thereof (hereafter collectively referred to as the active ingredients) may be administered by any route appropriate to the condition to be treated, suitable routes including oral, rectal, nasal, topical (including buccal and sublingual), vaginal and parenteral (including subcutaneous, intramuscular, intravenous, intradermal, intrathecal and epidural). It will be appreciated that the preferred route may vary with, for example, the condition of the recipient.

For each of the above-indicated utilities and indications the amount required of an active ingredient (as above defined) will depend upon a number of factors including the severity of the condition to be treated and the identity of the recipient and will ultimately be at the discretion of the attendant physician. In general, however, for each of these utilities and indications, a suitable, effective dose will be in the range 0.1 to 250 mg per kilogram body weight of recipient per day, preferably in the range 0.1 to 100 mg per kilogram body weight per day and most preferably in the range 1 to 20 mg per kilogram body weight per day; an optimum dose is about 15 mg per kilogram body weight per day (unless otherwise indicated all weights of active ingredient are calculated as the parent compound of formula (I); for salts and esters thereof the figures would be increased proportionately). The desired dose is preferably presented as two, three, four or more sub-doses administered at appropriate intervals throughout the day. These sub-doses may be administered in unit dosage forms, for example, containing 5 to 1000 mg, preferably 20 to 500 mg and most preferably 100 to 400 mg of active ingredient per unit dosage form.

While it is possible for the active ingredients to be administered alone it is preferable to present them as pharmaceutical formulations. The formulations of the present invention comprise at least one active ingredient, as above defined, together with one or more acceptable carriers thereof and optionally other therapeutic ingredients. The carrier (s) must be "acceptable" in the sense of being compatible with the other ingredients of the formulation and not deleterious to the recipients thereof.

The formulations include those suitable for oral, rectal,nasal, topical (including buccal and sublingual), vaginal or parenteral (including subcutaneous, intramuscular, intravenous, intradermal, intrathecal and epidural) administration. The formulations may conveniently be presented in unit dosage form and may be prepared by any of the methods well known in the art of pharmacy Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compression tablets may be prepared by compressing in an suitable machine the active ingredient in a free-flowing form such as a powder or granules, optionally mixed with a binder (e.g. povidone, gelatin, hydroxypropylmethyl cellulose), lubricant, inert diluent, preservative, disintegrant (e.g. sodium starch glycollate, cross-linked povidone, cross-linked sodium carboxymethyl cellulose), surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethylcellulose in varying proportions to provide the desired release profile.

For infections of the eye or other external tissues, e.g., mouth and skin, the formulations are preferably applied as a topical ointment or cream containing the active ingredient in an amount of, for example, 0.075 to 20% w/w, preferably 0.2 to 15% w/w and most preferably 0.5 to 10% w/w. When formulated in an ointment, the active ingredients may be employed with either a paraffinic or a water-miscible ointment base. Alternatively, the active ingredients may be formulated in a cream with an oil-in-water cream base.

If desired, the aqueous phase of the cream may include, for example, at least 30% w/w of a polyhydric alcohol, i. e. an alcohol having two or more hydroxyl groups such as propylene glycol, butane-1,3-diol, mannitol, sorbitol, glycerol and polyethylene glycol and mixtures thereof. The topical formulations may desirably include a compound which enhances absorption or penetration of the active ingredient through the skin or other affected areas. Examples of such dermal penetration enhancers include dimethylsulphoxide and related analogues.

The oily phase of the emulsions of this invention may be constituted from known ingredients in a known manner. While the phase may comprise merely an emulsifier (otherwise known as an emulgent), it desirably comprises a mixture of at least one emulsifier with a fat or an oil or with both a fat and an oil. Preferably a hydrophilic emulsifier is included together with an lipophilic emulsifier which acts as a stabilizer. It is also preferred to include both an oil and a fat. Together, the emulsifier(s) with or without stabilizer(s) make up the so-called emulsifying wax, and the wax together with the oil and/or fat make up the so-called emulsifying ointment base which forms the oily dispersed phase of the cream formulations.

Emulgents and emulsion stabilizers suitable for use in the formulation of the present invention include Tween 60, Span 80, cetostearyl alcohol, myristyl alcohol, glyceryl mono-stearate and sodium lauryl sulphate.

The choice of suitable oils or fats for the formulation is based on achieving the desired cosmetic properties, since the solubility of the active compound in most oils likely to be used in pharmaceutical emulsion formulations is very low Thus the cream should preferably be a non-greasy, non-staining and washable product with suitable consistency to avoid leakage from tubes or other containers. Straight or branched chain, mono-or dibasic alkyl esters such as di- isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils can be used.

Formulations suitable for topical administration to the eye also include eye drops wherein the active ingredient is dissolved or suspended in a suitable carrier, especially an aqueous solvent for the active ingredient. The active ingredient is preferably present in such formulations in a concentration of 0.5 to 20%, advantageously 0.5 to 10% particularly about 1.5% w/w.

Formulations suitable for topical administration in the mouth include lozenges comprising the active ingredient in a flavoured base, usually sucrose and acacia or tragacanth; pastilles comprising the active ingredient in an inert base such as gelatin and glycerin, or sucrose and acacia; and mouth-washes comprising the active ingredient in a suitable liquid carrier.

Formulations for rectal administration may be presented as a suppository with a suitable base comprising for example cocoa butter or a salicylate.

Formulations suitable for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable formulations wherein the carrier is a liquid, for administration as for example a nasal spray or as nasal drops, include aqueous or oily solutions of the active ingredient.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit daily sub-dose, as herein above recited, or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavouring agents.

The present invention also provides a process for the preparation of 9-[3-D-arabinofuranosyl-2-amino-6-methoxy-9H-purine comprising either:
A. reacting a compound of formula (II) 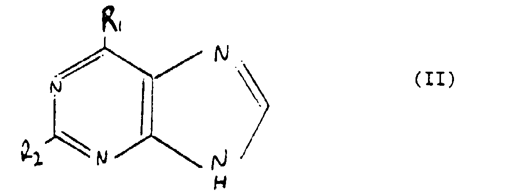 wherein R₁ is methoxy and R₂ is amino, with a compound of formula (III) 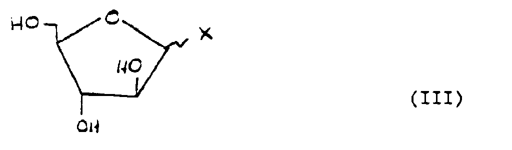 wherein X represents a pyrimidine or purine base (other than a compound of formula (II)); or
B. reacting a compound of formula (IV) 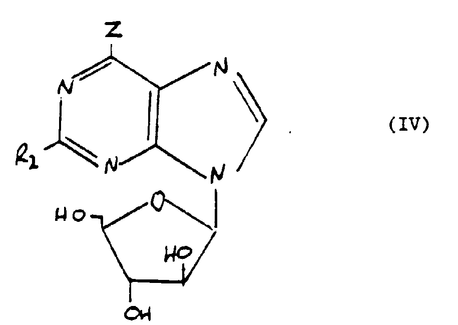 wherein Z is a leaving group and R₂ is amino, with a compound capable of introducing a methoxy group at the 6-position; and optionally thereafter or simultaneously therewith, where the resulting compound is a compound of formula (I), converting it into a pharmaceutically acceptable ester or ether thereof or where the resulting compound is a pharmaceutically acceptable ester or ether converting it into a different pharmaceutically acceptable ester or ether of or a compound of formula (I).

With regard to process A), X is advantageously a uracil base. The reaction may be effected, for example, by treatment of formulae II and III with an enzyme such as a phosphorylase enzyme, for example, uridine phosphorylase or purine nucleoside phosphorylase, or a mixture thereof preferably in the presence of a phosphate salt at an pH of 5.0-9.0 and a temperature of 15°-90°C advantageously 40°-60°C.

Regarding process B), this this may be carried out in accordance with the procedure described by Reist. E.J. et al., J. Org. Chem. 27 (1962) 3274-3279. A convenient leaving group is a halogen atom for example chlorine, the reaction being advantageously carried out in an organic solvent, e.g. absolute methanol, with an agent capable of providing the necessary group at the 6-position for example, an appropriate amine in the case where R₁ represents an alkyl- or dialkylamino group

Physiologically acceptable esters and salts of the compounds of formula (I) may be prepared in conventional manner for example esters may be prepared by esterification of the parent compound with an appropriate acyl halide or anhydride. Alternatively the esters may be prepared by displacing an appropriate leaving group e.g. halide with an appropriate carboxylic acid or by opening an appropriate anhydro nucleoside of the parent compound with an appropriate carboxylic acid or salt thereof.

The following Examples illustrate the present invention but should not be considered in any way limiting of the invention.

### Example 1 : 9-(β-D-Arabinofuranosyl-6-methoxypurine

6-Methoxypurine (Sigma Chemical Co. St. Louis, MO), (6.6 mmoles, Ig), and uracil arabinoside (Torrence, P.F. et al J. Med.Chem, 22(3) (1979)) (10.1 mmoles, 2.45g) were suspended in 575ml of a 10 mM potassium phosphate, 0.04% potassium azide solution, pH of 7.8, containing 10% n-propanol (v/v). Purified uridine phosphorylase (560 I.U.) and purine nucleoside phosphorylase (10000 l.U.) (Krenitsky, T.A. et al, Biochemistry, 20, 3615, 1981 and US Patent 4,381,444) were added and the solution stirred at 35°C. Thirty days later the reaction was filtered. The filtrate was adjusted to pH 10.5 with ammonium hydroxide and chromatographed on a 2.5 x 7cm column containing Dowex-1-formate resin. The resin was eluted with 30% n-propanol/water (v/v). Fractions containing the product were combined and the solvent removed under vacuum. The residue was dissolved in 30% n-propanol/water (v/v) and chromatographed on BioRad P-2 column (7.5 x 90 cm). Product containing fractions were combined and after lyophilization yielded 0.922 g of 6-methoxypurine-9-p-D-arabinofuranoside as a dihydrate.

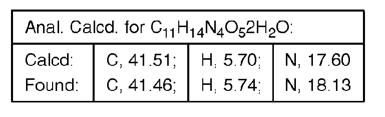

NMR and mass spectrometry were consistent with the structure.

### Example 2 : 9-(β-D-Arabinofuranosyl-2-amino-6-methoxypurine

2-Amino-6-methoxypurine (prepared by nucleophilic displacement of the chlorine group on 2-amino-6-chloropurine (Sigma Chemicals St. Louis MO) by methanol with sodium hydride in tetrahydrofuran)(6.4mmol, 1.05g) was combined with 35ml of a uracil arabinoside solution (7.04mmol, 1.75g) in 10 mM potassium phosphate and 7% n-propanol (v/v). The pH was adjusted to 6.75. Purified purine nucleoside phosphorylase (18000 units) and uridine phosphorylase (1020 units) were added and the solution incubated at 37°C. After 26 days the reaction was filtered and the filtrate chromatographed on a column of Dowex-1-formate resin (2 x 7cm) after adjusting the pH to 10.5 with concentrated ammonium hydroxide. The column was eluted with 7% n-propanol/water (v/v) and fractions containing product were combined and solvent removed in vacuo. The residue was extracted with 25ml of water and the filtrate separated from the solids by centrifugation. The supernatant upon standing at ambient temperature formed crystals of 2-amino-6-methoxypurine -9-p-D-arabinofuranoside which after drying in vacuo, yielded 0.327g of product.

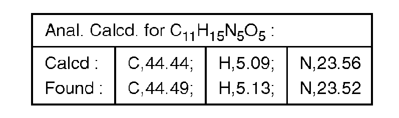

NMR and mass spectrometry were consistent with the structure.

### Example 3:

### 9-(5-O-(4-Methylbenzoyl)-[β-D-arabinofuranosyl)-6-methoxy-9H-purine

9-((β-D-Arabinofuranosyl)-6-methoxy-9H-purine from Example 1 (0.283g, 1.0 mmole) was suspended in anhydrous acetonitrile (10.0 ml), pyridine (2.3 ml) was added to effect complete solution, followed by 4-methyl-benzoyl chloride (0.170g, 1.1 mmole). The mixture was stirred at room temperature for 24 hours under argon, quenched by adding isopropanol (5ml), and evaporating to dryness, followed by coevaporation with ethanol (2 x 10 ml). The residue was then purified by flash chromatography on silica gel (25.0g, 2.5 x 15 cm) with a stepped gradient of CHCl₃ to 1:1 CHCl₃ : acetone. Those fractions containing product with an R_{f}-0.41 (silica gel, CHCl₃:acetone/ 1:1 ) were combined to afford 132 mg of the desired compound :
mp : 127-128°C;
Analysis calculated for C₁₉H₂₀N₄O₆ . 0.5 H₂O:C,55.76; H,5.17;N,13.69.
Found: C,55.48; H,5.36; N,13.64.
NMR and mass spectrometry were consistent with the structure.

### Example 4 :

### 9-5-O-(6-Nitrobenzoyl)-[β-D-arabinofuranosyl)-6-methoxy-9H-purine

9-([β-D-Arabinofuranosyl)-6-methoxy-9H-purine from Example 1 (0.283g, 1.0mmole) was suspended in anhydrous acetonitrile (10.0ml), pyridine (2.3ml) was added to effect complete solution, followed by 4-nitro-benzoyl chloride (0.205g, 1.1 mmole). The mixture was stirred at room temperature for 24 hours under argon, quenched by adding isopropanol (5ml), and evaporating to dryness, followed by coevaporation with ethanol (2x1 Oml). The residue was then purified by flash chromatography on silica gel (25.0g, 2.5 x 15cm) with a stepped gradient of CHCI₃ to CHCI₃ : acetone (1:1). Those fractions containing product with an R_{f}=0.37 (silica gel, CHCl₃:acetone (1:1)) were combined to afford 105mg of the desired compound :
mp : 202-203°C;
Analysis calculated for C₁₈H₁₇N₅O₈: C,50.12; H,3.97; N,16.24.
Found : C,50.21, H,4.02; N,16.16.
NMR and mass spectrometry were consistent with the structure.

### Example 5 :

### 9-[5-O-(4-Aminobenzoyl-β-D-arabinofuranosyl))-6-methoxy-9H-purine

9-(5-0-(4-Nitrobenzoyl)-(β-D-arabinofuranosyl)-6-methoxy-9H-purine from Example 4 (0.350g, 0.81 mmole) was suspended in ethanol (100.0ml) and 10% palladium-on-carbon (0.100g) was added. After alternately evacuating and charging the system with hydrogen, the reaction is shaken on a Parr apparatus at 50 psi for three hours. The mixture is then filtered through Celite and the filtrate evaporated to dryness. The residue after evaporation was suspended in methanol and filtered to afford the desired compound as a white solid (0.285g, 88%) :
mp: 198-200°C;
Analysis calculated for C₁₈H₁₉N₅O₆ . 0.3 H₂O : C,53.15; H,4.86; N,17.22.
Found : C,52.96; H,4.64; N,17.07.
NMR and mass spectrometry were consistent with the structure.

### Example 6 : 6-Methoxy-9-(2-O-pentanoyl-(β-D-arabinofuranosyl)-9H-purine Method 1

9-([β-D-Arabinofuranosyl)-6-methoxy-9H-purine from Example 1 (283mg, 1.0 mmole) acetonitrile (20ml) and pyridine (5ml) were added to a three-necked round bottom flask equipped with thermometer, argon inlet valve, and magnetic stirrer. Valeric anhydride (0.2ml, 1.0mmole) was added and the solution allowed to stir at 20°C for three hours. The reaction was quenched with water (2ml) and evaporated to a clear oil, then purified by flash chromatography on silica with a stepped gradient of CHC1₃ to CHCI₃:MeOH (9: 1). This yielded 110 mg of the 2'-ester contaminated with the 5'- ester, a lower R_{f} material. This material was further purified by preparative layer chromatography on silica with CHCl₃: MeOH(9:1 ) and co-evaporated with acetone to afford 70mg of the 2'-ester as a clear glass.
Analysis calculated for C₁₆H₂₂N₄O₆. 0.5 (CH₃)₂CO : C,53.16; H,6.37; N,14.17.
Found : C,52.89; H,6.37; N,13.96.
NMR and mass spectrometry were consistent with the structure.

### Method 2

6-Methoxy-9-[3,5-O-(1;1,3,3-tetraisopropyldisiloxan-1,3-diyl)-[β-D-arabinofuranosyl]-9H-purine from Example 7 (2.5g, 4.8 mmol) was added to a 250ml round bottom flask along with 4-dimethylaminopyridine (0.06g, 0.47 mmol). Dry acetonitrile (30ml) and triethylamine (2.65ml) were added, and a constant addition funnel was charged with acetonitrile (20ml) and pentanoic anhydride (1.13ml, 5.7mmol). The reaction mixture was cooled in an 3°C ice bath under a flow of argon. Slow addition of the pentanoic anhydride solution was carried out over a period of 2 hours. The reaction mixture was then treated with methanol (10ml) and concentrated under reduced pressure. The residue was taken up in CHC1₃ (200ml) and extracted with H₂0 (2x50ml). The combined aqueous layers were back extracted with CHCl₃ (25ml) and the combined organic layers were dried (MgS0₄), filtered, and concentrated to give 3.44 gms of crude product.

The crude 6-methoxy-9-[2-O-pentanoyl-3,5-O-(1,1,3,3-tetraisopropyldisiloxan -1,3-diyl)-[β-D-arabinofuranosyl]-9H-purine was dissolved in tetrahydrofuran (120ml) and H₂0 (3ml). The solution was cooled to 3°C in an ice bath and a 1.0M solution of tetrabutylammonium fluoride in tetrahydrofuran (6.0ml) was added. After 1.5 hours, a saturated ammonium chloride solution (2ml) was added and the reaction mixture was passed directly onto a silica gel scrub column (5.0 x 8.0 cm) packed in chloroform. The column was eluted with chloroform (200ml) then with 1:1 acetone: chloroform (400ml) and all the product containing fractions were combined and concentrated. Final purification was accomplished on a silica gel flash column (5.0x15cm) eluted with a stepped gradient of acetone in CHCl₃ providing 1.17g (67%) of a clear sticky glass contaminated with pentanoic acid:
Analysis calculated for C₁₆H₂₂N₄O₆ 0.2 C₅H₁₀O₂: C,52.79; H,6.25; N,14.48.
Found: C,52.97; H,6.38; N,14.60.

### Example 7 :

### 6-Methoxy-9-[3,5-O-(1,2,3, 3-tetraisopropyl-1, 3-disiloxan-1,3-diyl)-(β-D-arabinofuranosyl)-9H-pu rine

9-([β-D-Arabinofuranosyl)-6-methoxy-9H-purine from Example 3 (1.0g, 3.54mmol) and imidazole (0.965g, 14.2mmol) were dissolved in dry dimethylformamide (10ml) and the solution was chilled to 3°C. 1,3-Dichloro-1,1,3,3-tetraisopropyldisiloxane (1.35ml, 3.90mmol) was then added and the mixture stirred under argon for three hours. The reaction mixture was then quenched with H₂0 (1 ml) and concentrated under reduced pressure. The residue was partitioned between ethyl acetate (150ml) and H₂0 (50ml), and the ethyl acetate layer dried (MgS0₄), filtered and concentrated. The crude product was taken up in ethyl acetate and purified by flash chromatography on silica gel (25.0g, 2.5 x 15cm) in ethyl acetate. Those fractions with an R_{f} of 0.70 (silica, ethyl acetate) provided 1.48g (80%) of the desired material as a white solid.
UV max : EtOH : 248.4nm.
NMR and mass spectrometry were consistent with the structure.

### Example 50 : Tablet Formulations

The following formulations A and B are prepared by wet granulation of the ingredients with a solution of povidone, followed by addition of magnesium stearate and compression.

### Formulation A

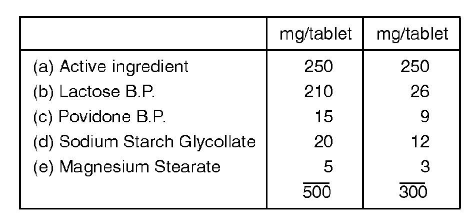

### Formulation B

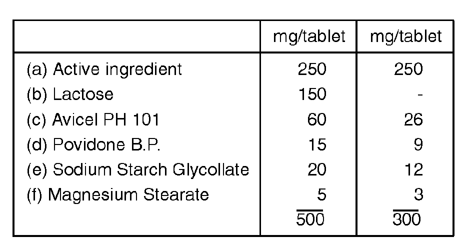

### Formulation C

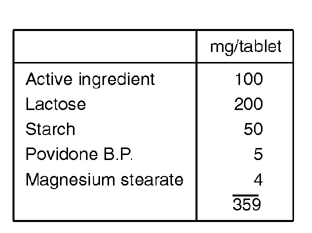

Tablets are prepared from the foregoing ingredients (C) by wet granulation followed by compression. In an alternative preparation the Povidone B.P. may be replaced by Polyvinylpyrrolidone.

The following formulations, D and E, are prepared by direct compression of the admixed ingredients. The lactose in formulation E is of the direct compression type (Dairy Crest - "Zeparox").

### Formulation D

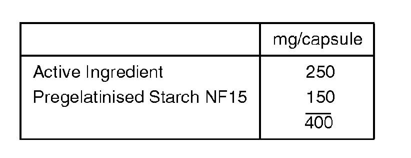

### Formulation E

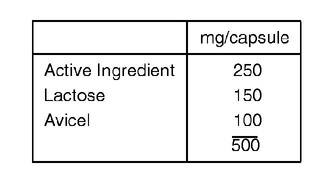

### Formulation F (Controlled Release Formulation)

The formulation is prepared by wet granulation of the ingredients (below) with a solution of povidone followed by the addition of magnesium stearate and compression.

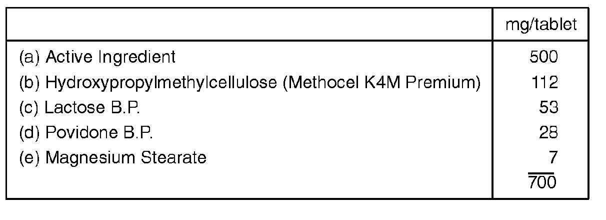

Drug release takes place over a period of about 6-8 hours and is complete after 12 hours.

### Example 51 : Capsule Formulations

### Formulation A

A capsule formulation is prepared by admixing the ingredients of Formulation D in Example 50 above and filling into a two-part hard gelatin capsule. Formulation B (infra) is prepared in a similar manner.

### Formulation B

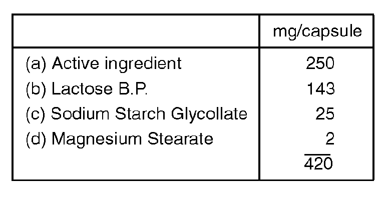

### Formulation C

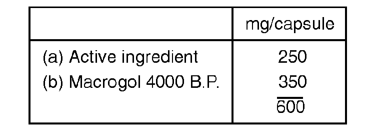

Capsules are prepared by melting the Macrogol 4000 BP, dispersing the active ingredient in the melt and filling the melt into a two part hard gelatin capsule.

### Formulation D

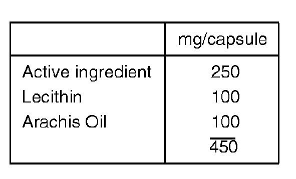

Capsules are prepared by dispersing the active ingredient in the lecithin and arachis oil and filling the dispersion into soft, elastic gelatin capsules.

### Formulation E (Controlled Release Capsule)

The following controlled release capsule formulation is prepared by extruding ingredients a, b and c using an extruder, followed by spheronisation of the extrudate and drying. The dried pellets are then coated with release-controlling membrane (d) and filled into a two-piece, hard gelatin capsule.

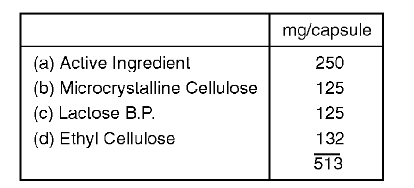

### Example 52 : Ophthalmic Solution

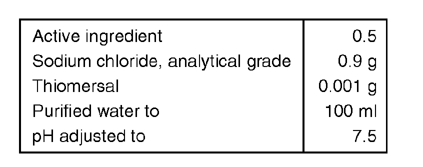

### Example 53 : Injectable Formulation

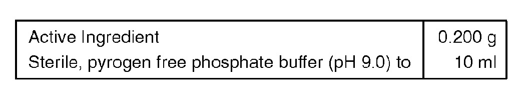

The active ingredient is dissolved in most of the phosphate buffer (35°-60°C), then made up to volume and filtered through a sterile micropore filter into a sterile 10ml amber glass vial (type 1) and sealed with sterile closures and overseals.

### Example 54 : Intramuscular injection

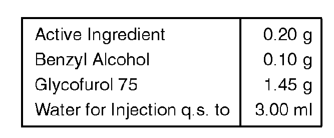

The active ingredient is dissolved in the glycofurol. The benzyl alcohol is then added and dissolved, and water added to 3 ml. The mixture is then filtered through a sterile micropore filter and sealed in sterile 3 ml amber glass vials (type 1

### Example 55 : Syrup Suspension

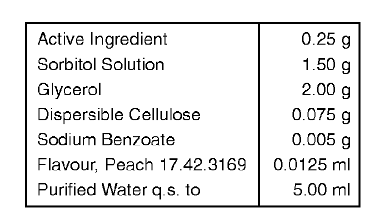

The sodium benzoate is dissolved in a portion of the purified water and the sorbitol solution added. The active ingredient is added and dispersed. In the glycerol is dispersed the thickener (dispersible cellulose). The two dispersions are mixed and made up to the required volume with the purified water. Further thickening is achieved as required by extra shearing of the suspension.

### Example 56 : Suppository

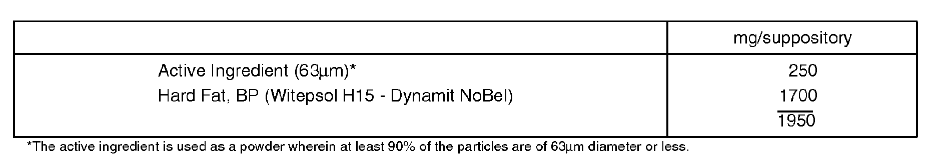

One-fifth of the Witepsol H15 is melted in a steam-jacketed pan at 45°C maximum. The active ingredient is sifted through a 200pm sieve and added to the molten base with mixing, using a silverson fitted with a cutting head, until a smooth dispersion is achieved. Maintaining the mixture at 45°C, the remaining Witepsol H15 is added to the suspension and stirred to ensure a homogenous mix. The entire suspension is passed through a 250µm stainless steel screen and, with continuous stirring, is allowed to cool to 40°C. At a temperature of 38°C to 40°C, 2.02g of the mixture is filled into suitable plastic moulds. The suppositories are allowed to cool to room temperature.

### Example 57 : Pessaries

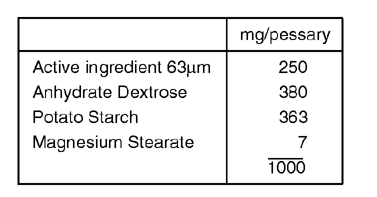

The above ingredients are mixed directly and pessaries prepared by direct compression of the resulting mixture.

### Example 58 : Antiviral Toxicity & Bioavailability Testing Determination of Anti-Varicella-Zoster Virus Activity:

The inhibitory effects of compounds on the replication of VZV (Oka strain) were assessed by an ELISA procedure (Berkowitz, F.E., and Levin, M.J. (1985). Antimicrob. Agents and Chemother. 28: 207-210) that was modified as follows. Infections were initiated in the presence of drug, rather than before drug addition. At the end of the three day incubation of drug and virus with uninfected cells (human diploid fibroblasts, strain MRC-5), the 96-well plates were centrifuged for 5 minutes at 200 x g to sediment detached cells prior to glutaraldehyde fixation. The present ELISA used an alkaline phosphatase-conjugated anti-human IgG as the second antibody. The rate of cleavage of p-nitrophenyl phosphate by bound alkaline phosphatase was determined as described elsewhere (Tadepalli, S.M., Quinn, R.P., and Averett, D.R. 1986. Antimicrob. Agents and Chemother. 29: 93-98). Uninfected cells were used to obtain the blank reaction rates, which were subtracted from the rates obtained with the virus present. This assay was suitable to detect progeny virus in cultures that were initially infected with 15 to 3600 infectious particles per well.

### Determination of Growth Inhibition of Uninfected Mammalian Cells

The capability of candidate compounds to inhibit the growth of D98 cells (human) and L cells (murine) was measured by determination of cell number following three days exposure of a standard number of cells to various dilutions of compound (Rideout, J. L., Krenitsky, T.A., Koszalka, G.W., Cohn, N.K., Chao, E.Y. Elion, G.B., Latter, V.S., and Williams, R.B. (1982) J. Med Chem. 25: 1040-1044). The cell number was then compared to the number obtained in the absence of compound. Cell enumeration was performed by either direct particle counts following trypsinization of the monolayer, or by spectrophotometric determination of the amount of vital stain taken up by the cells. Comparable results were obtained with both methods.

### Data Analysis

The concentration of compound resulting in 50% of control values (IC50) was calculated either by direct interpolation from graphs of the log of the compound concentration versus the percent of control value, or from a computer program which analyses the data according to the same algorithm. Data in the range of 20% to 80% of control were used in these calculations.

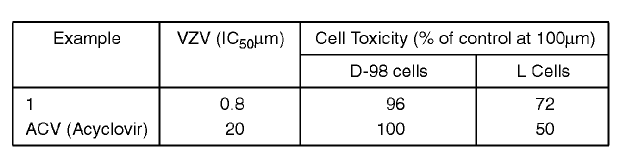

### Bioavailability Assay & Data

Two Long-Evans rats were dosed with the compound by gavage with an intragastric tube at a dose of 1 Omg/kg of Example 1 or the molar equivalent of Example 3, 5 and 6. The animals were housed in metabolic cages and urine was collected for the 0-24 hour and 24-48 hour periods post dose. The urines were analysed by reverse-phase HPLC. Results are expressed as % Dose recovered in the urine in 48 hours as compound of Example 1.

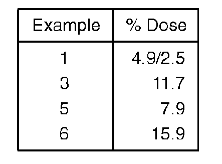

## Claims (Claims for the following Contracting State(s) : AT, BE, CH, DE, FR, GB, IT, LI, LU, NL, SE)

1. The use of compound of formula (I) 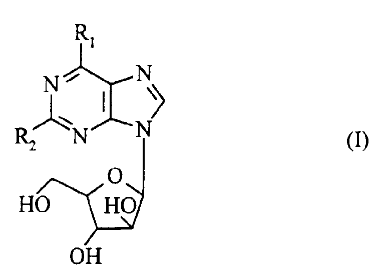 wherein R₁ is methoxy and R₂ is hydrogen or amino, and any pharmaceutically acceptable salts, ethers, esters or salts of such esters in the manufacture of a medicament for the treatment or prophylaxis of human viral infections caused by VZV.

2. 9-p-D-Arabinofuranosyl-2-amino-6-methoxy-9H-purine or a physiologically acceptable derivative thereof.

3. 9-[β-D-Arabinofuranosyl-2-amino-6-methoxy-9H-purine.

4. 9-(β-D-Arabinofuranosyl-2-amino-6-methoxy-9H-purine for use in medicine.

5. A pharmaceutically acceptable ester of a compound of formula (I) selected from:-
9-(5-O-(4-methylbenzoyl)-[3-D-arabinofuranosyl)-6-methoxy-9H-purine;
9-(5-O-(4-Aminobenzoyl)-[β-D-arabinofuranosyl)-6-methoxy-9H-purine; or
6-methoxy-9-(2-O-pentanoyl-[β-D-arabinofuranoyl)-9H-purine.

6. A process for the preparation of 9-p-D-Arabinofuranosyl-2-amino-6-methoxy-9H-purine or a salt, ether, ester or salt of such ester comprising either:
a) reacting a compound of formula (II) 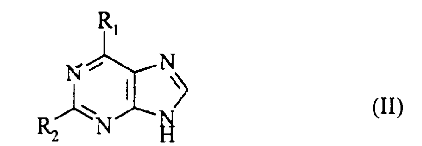 wherein R₁ is methoxy and R₂ is amino, with a compound of formula (III) 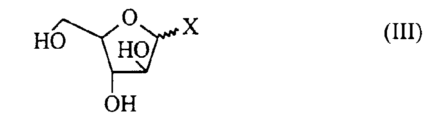 wherein X represents a pyrimidine or purine base (other than a compound of formula (II); or
b) reacting a compound of formula (IV) 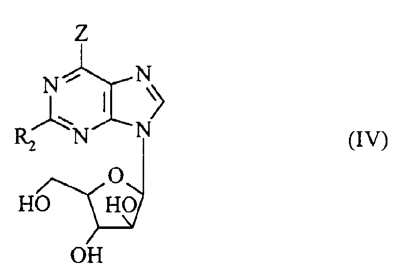 wherein Z is a leaving group and R₂ is amino, with a compound capable of introducing a methoxy group at the 6-position; and optionally thereafter or simultaneously therewith, wherein the resulting compound is 9-p-D-Arabinofuranosyl-2-amino-6-methoxy-9H-purine, converting it into a pharmaceutically acceptable salt, ether, ester or salt of such ester or where the resulting compound is a pharmaceutically acceptable salt, ether, ester or salt of such ester converting it into a different pharmaceutically acceptable salt, ether, ester or salt of such ester of 9-p-D-Arabinofuranosyl-2-amino-6-methoxy-9H-purine.

7. A pharmaceutical formulation comprising 9-p-D-Arabinofuranosyl-2-amino-6-methoxy-9H-purine or a salt, ether, ester or salt of such ester, together with a pharmaceutically acceptable carrier.

8. A pharmaceutical formulation as claimed in claim 7 wherein the active is 9-p-D-Arabinofuranosyl-2-amino-6-methoxy-9H-purine.

9. A formulation as claimed in claims 7 or 8 in unit dosage form wherein the unit dosage of active compound is 5 to 1000 mg.

10. A formulation as claimed in claims 7, 8 or 9 adapted for oral administration.

11. A formulation as claimed in claim 10 in the form of a capsule, cachet or tablet.

## Claims (Claims for the following Contracting State(s) : ES, GR)

1. A process for the preparation of 9-p-D-Arabinofuranosyl-2-amino-6-methoxy-9H-purine or a salt, ether, ester or salt of such ester comprising either:
a) reacting a compound of formula (II) 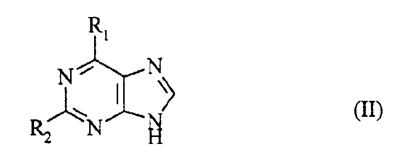 wherein R₁ is methoxy and R₂ is amino, with a compound of formula (III) 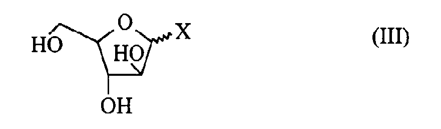 wherein X represents a pyrimidine or purine base (other than a compound of formula (II); or
b) reacting a compound of formula (IV) 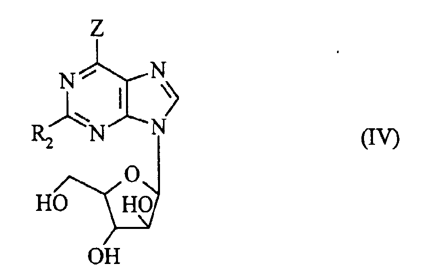 wherein Z is a leaving group and R₂ is amino, with a compound capable of introducing a methoxy group at the 6-position; and optionally thereafter or simultaneously therewith, wherein the resulting compound is a compound of formula (I), converting it into a pharmaceutically acceptable salt, ether, ester or salt of such ester or where the resulting compound is a pharmaceutically acceptable salt, ether, ester or salt of such ester converting it into a different pharmaceutically acceptable salt, ether, ester or salt of such ester of a compound of formula (I).

2. A process as claimed in claim 1 wherein 9-β-D-Arabinofuranosyl-2-amino-6-methoxy-9H-purine is prepared.

3. A process as claimed in claim 2 wherein the pharmaceutically acceptable ester is selected from:-
9-(5 -O-(4-methylbenzoyl)-β-D-arabinofuranosyl)-6-methoxy-9H-purine;
9-(5-O-(4-Aminobenzoyl)-β-D-arabinofuranosyl)-6-methoxy-9H-purine; or
6-methoxy-9-(2-O-pentanoyl-β-D-arabinofuranoyl)-9H-purine.

4. A method of preparing a medicament which comprises:
a) preparing 9-β-D-Arabinofuranosyl-2-amino-6-methoxy-9H-purine or a salt, ether, ester or salt of such ester by a process as claimed in claim 1 to 3, and
b) bringing into association the product from step a) with one or more pharmaceutically acceptable carriers and, optionally, one or more other physiologically active agents.

5. A method as claimed in claim 4 which comprises the additional step of forming the admixture of step b) into a tablet or capsule.

## Patentansprüche (Patentansprüche für folgende(n) Vertragsstaat(en) : AT, BE, CH, DE, FR, GB, IT, LU, LI, NL, SE)

1. Verwendung einer Verbindung der Formel (I) 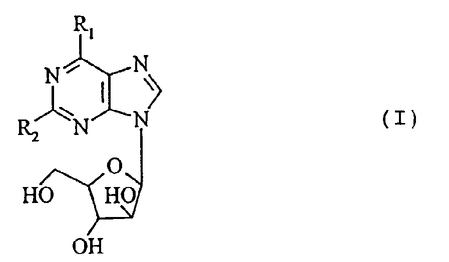 worin R₁ Methoxy und R₂ Wasserstoff oder Amino bedeutet, und irgendwelcher pharmazeutisch annehmbarer Salze, Ether, Ester oder Salze solcher Ester zur Herstellung eines Arzneimittels für die Behandlung oder Prophylaxe von humanen viralen Infektionen, die durch VZV verursacht werden.

2. 9-ß-D-Arabinofuranosyl-2-amino-6-methoxy-9H-purin oder ein physiologisch annehmbares Derivat davon.

3. 9-β-D-Arabinofuranosyl-2-amino-6-methoxy-9H-purin.

4. 9-ß-D-Arabinofuranosyl-2-amino-6-methoxy-9H-purin für die Verwendung in der Medizin.

5. Pharmazeutisch annehmbarer Ester einer Verbindung der Formel (I), ausgewählt aus:
9-(5-O-(4-Methylbenzoyl)-β-D-arabinofuranosyl)-6-methoxy-9H-purin;
9-(5-O-(4-Aminobenzoyl)-β-D-arabinofuranosyl)-6-methoxy-9H-purin; oder
6-Methoxy-9-(2-O-pentanoyl-ß-D-arabinofuranoyl)-9H-purin.

6. Verfahren zur Herstellung von 9-ß-D-Arabinofuranosyl-2-amino-6-methoxy-9H-purin oder einem Salz, Ether, Ester oder einem Salz eines solchen Esters, dadurch gekennzeichnet, daß entweder
a) eine Verbindung der Formel (II) 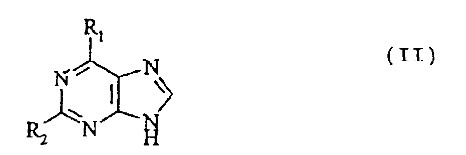 worin R₁ Methoxy und R₂ Amino bedeuten, mit einer Verbindung der Formel (III) 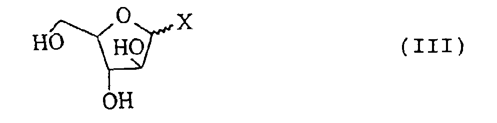 worin X eine Pyrimidin- oder Purinbase (ausgenommen einer Verbindung der Formel (11)) bedeutet, umgesetzt wird; oder
b) eine Verbindung der Formel (IV) 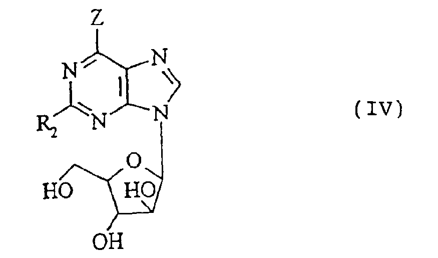 worin Z eine Austrittsgruppe und R₂ Amino bedeuten, mit einer Verbindung, welche eine Methoxygruppe in 6-Stellung einführen kann, umgesetzt wird, und gegebenenfalls danach oder gleichzeitig damit, wenn die entstehende Verbindung ein 9-β-D-Arabinofuranosyl-2-amino-6-methoxy-9H-purin ist, diese in ein pharmazeutisch annehmbares Salz, einen Ether einen Ester oder ein Salz eines solchen Esters überführt wird, oder wenn die entstehende Verbindung ein pharmazeutisch annehmbares Salz, ein Ether, ein Ester oder ein Salz eines solchen Esters ist, diese in ein unterschiedliches pharmazeutisch annehmbares Salz, einen Ether, einen Ester oder ein Salz eines solchen Esters von 9-β-D-Arabinofuranosyl-2-amino-6-methoxy-9H-purin überführt wird.

7. Pharmazeutische Zubereitung, enthaltend 9-ß-D-Arabinofuranosyl-2-amino-6-methoxy-9H-purin oder ein Salz, einen Ether, einen Ester oder ein Salz eines solchen Esters zusammen mit einem pharmazeutisch annehmbaren Träger.

8. Pharmazeutische Zubereitung nach Anspruch 7, worin der aktive Bestandteil 9-ß-D-Arabinofuranosyl-2-amino-6-methoxy-9H-purin ist.

9. Zubereitung nach Anspruch 7 oder 8 in Dosiseinheitsform, worin die Einheitsdosis an aktiver Verbindung 5 bis 1000 mg beträgt.

10. Zubereitung nach einem der Ansprüche 7, 8 oder 9, angepaßt für die orale Verabreichung.

11. Zubereitung nach Anspruch 10 in Form einer Kapsel, eines Kachets oder einer Tablette.

## Patentansprüche (Patentansprüche für folgende(n) Vertragsstaat(en) : ES, GR)

1. Verfahren zur Herstellung von 9-ß-D-Arabinofuranosyl-2-amino-6-methoxy-9H-purin oder eines Salzes, Ethers, Esters oder Salzes eines solchen Esters, dadurch gekennzeichnet, daß entweder:
a) eine Verbindung der Formel (11) 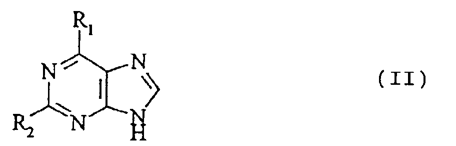 worin R₁ Methoxy und R₂ Amino bedeuten, mit einer Verbindung der Formel (III) 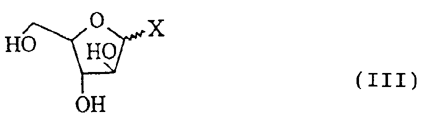 worin X eine Pyrimidin- oder Purinbase bedeutet (ausgenommen einer Verbindung der Formel (II)) umgesetzt wird, oder
b) eine Verbindung der Formel (IV) 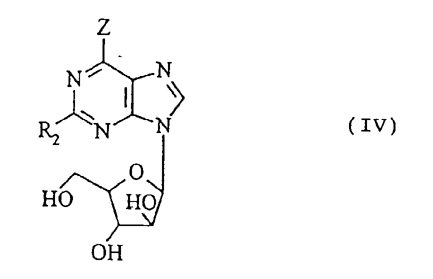 worin Z eine Austrittsgruppe und R₂ Amino bedeutet, mit einer Verbindung, die eine Methoxygruppe in die 6-Stellung einführen kann, umgesetzt wird, und gegebenenfalls danach oder gleichzeitig damit, wenn die entstehende Verbindung eine Verbindung der Formel (I) ist, diese in ein pharmazeutisch annehmbares Salz, einen Ether, einen Ester oder ein Salz eine solchen Esters überführt wird, oder wenn die entstehende Verbindung ein pharmazeutisch annehmbares Salz, ein Ether, ein Ester oder ein Salz eines solchen Esters ist, diese in ein unterschiedliches pharmazeutisch annehmbares Salz, einen Ether, einen Ester oder ein Salz eines solchen Esters einer Verbindung der Formel (I) umwandelt.

2. Verfahren nach Anspruch 1, wobei 9-ß-D-Arabinofuranosyl-2-amino-6-methoxy-9H-purin hergestellt wird.

3. Verfahren nach Anspruch 2, worin der pharmazeutisch annehmbare Ester ausgewählt wird aus:
9-(5-O-(4-Methylbenzoyl)-β-D-arabinofuranosyl)-6-methoxy-9H-purin;
9-(5-O-(4-Aminobenzoyl)-β-D-arabinofuranosyl)-6-methoxy-9H-purin; oder
6-Methoxy-9-(2-O-pentanoyl-ß-D-arabinofuranoyl)-9H-purin.

4. Verfahren zur Herstellung eines Arzneimittels, welches
a) die Herstellung von 9-ß-D-Arabinofuranosyl-2-amino-6-methoxy-9H-purin oder eines Salzes, Ethers, Esters oder Salz eines solchen Esters gemäß einem Verfahren nach einem der Ansprüche 1 bis 3, und
b) Zusammenbringen in Assoziierung des Produktes der Stufe a) mit einem oder mehreren pharmazeutisch annehmbaren Trägern und gegebenenfalls einem oder mehreren physiologisch aktiven Mitteln umfaßt.

5. Verfahren nach Anspruch 4, welches die zusätzlichen Stufen umfaßt, daß das Gemisch der Stufe b) in eine Tablette oder Kapsel überführt wird.

## Revendications (Revendications pour l'(les) Etat(s) contractant(s) suivant(s) : AT, BE, CH, DE, FR, GB, IT, LU, LI, NL, SE)

1. Utilisation du composé de la formule (I) 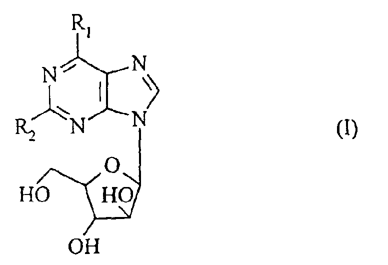 dans laquelle R₁ représente le radical méthoxy et R₂ représente un atome d'hydrogène ou le radical amino et de n'importe quels sels, éthers, esters ou sels de tels esters, pharmaceutiquement acceptables, pour la fabrication d'un médicament destiné au traitement ou à la prophylaxie d'infections virales humaines provoquées par le VZV

2. 9-(β-D-Arabinofurannosyl-2-amino-6-méthoxy-9H-purine ou dérivé physiologiquement acceptable de celle-ci.

3. 9-(β-D-Arabinofurannosyl-2-amino-6-méthoxy-9H-purine.

4. 9-[β-D-Arabinofurannosyl-2-amino-6-méthoxy-9H-purine en vue de son utilisation en médecine.

5. Ester pharmaceutiquement acceptable d'un composé de la formule (I) choisi parmi les substances qui suivent :
9-(5-O-(4-Méthylbenzoyl)-[β-D-arabinofurannosyl)-6-méthoxy-9H-purine;
9-(5-O-(4-Aminobenzoyl)-β-D-arabinofurannosyl)-6-méthoxy-9H-purine et
6-Méthoxy-9-(2-O-pentanoyl-β-D-arabinofurannosyl)-9H-purine.

6. Procédé de préparation de la 9-β-D-arabinofurannosyl-2-amino-6-méthoxy-9H-purine, caractérisé en ce que :
A. On fait réagir un composé de la formule (II) 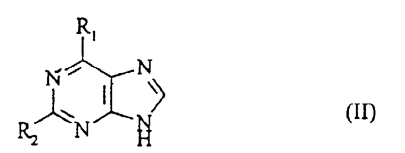 dans laquelle Rᵢ représente le radical méthoxy et R₂ représente le radical amino, avec un composé de la formule (III) 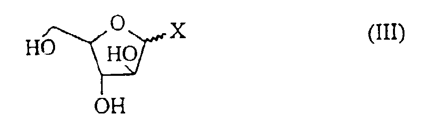 dans laquelle X représente une base du type pyrimidine ou purine (autre qu'un composé de la formule (II), ou bien
B. On fait réagir un composé de la formule (IV) 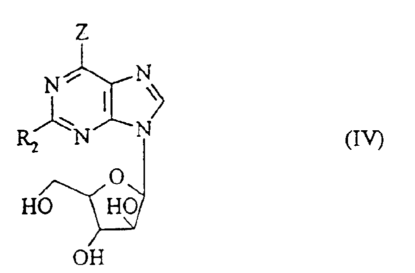 dans laquelle Z représente un groupe sortant ou labile et R₂ représente le radical amino, avec un composé capable d'introduire le radical méthoxy en position 6 et éventuellement ensuite ou simultanément à cette réaction, lorsque le composé obtenu est la 9-β-D-arabinofurannosyl-2-amino-6-méthoxy-9H-purine, on le convertit en un sel, éther, ester ou sel d'un tel ester, ou lorsque le composé résultant est un sel, éther, ester ou sel d'un tel ester pharmaceutiquement acceptable, on le convertit en un sel, éther, est ou sel d'un tel ester de pharmaceutiquement acceptable, dfférent de la 9-β-D-arabinofurannosyl-2-amino-6-méthoxy-9H-purine.

7. Composition pharmaceutique comprenant de la 9-β-D-arabinofurannosyl-2-amino-6-méthoxy-9H-purine ou un sel, éther, ester ou sel d'un tel ester, en même temps qu'un véhicule ou excipient pharmaceutiquement acceptable.

8. Composition pharmaceutique suivant la revendication 7, caractérisée en ce que l'ingrédient actif est la 9-p-D-arabinofurannosyl-2-amino-6-méthoxy-9H-purine.

9. Composition suivant la revendication 7 ou 8 sous forme de dose unitaire où la dose unitaire de composé actif varie de 5 à 1000 mg.

10. Composition suivant l'une quelconque des revendications 7, 8 et 9, convenant à l'administration par la voie orale.

11. Composition suivant la revendication 10, caractérisée en ce qu'elle se présente sous la forme d'une gélule, d'un cachet, ou d'un comprimé.

## Revendications (Revendications pour l'(les) Etat(s) contractant(s) suivant(s) : ES, GR)

1. Procédé de préparation de la 9-β-D-arabinofurannosyl-2-amino-6-méthoxy-9H-purine ou un sel, éther, ester ou un sel d'un tel ester, caractérisé en ce que :
A. On fat réagir un composé de la formule (II) 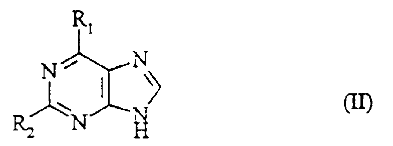 dans laquelle Rᵢ représente le radical méthoxy et R₂ représente le radical amino, avec un composé de la formule (III) 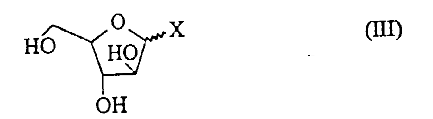 dans laquelle X représente une base du type pyrimidine ou purine (autre qu'un composé de la formule (II), ou bien
B. On fait réagir un composé de la formule (IV) 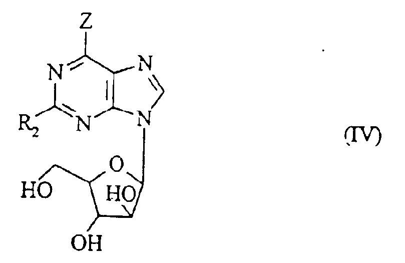 dans laquelle Z représente un groupe sortant ou labile et R₂ représente le radical amino, avec un composé capable d'introduire le radical méthoxy en position 6 et éventuellement ensuite ou simultanément à cette réaction, lorsque le composé obtenu est un composé de la formule (1), on le convertit en un sel, éther, ester ou un sel d'un tel ester pharmaceutiquement acceptable, ou lorsque le composé résultant est un sel, éther, ester ou un sel d'un tel ester pharmaceutiquement acceptable, on le convertit en un sel, éther, ester ou un sel d'un tel ester pharmaceutiquement acceptable différent, ou un composé de la formule (1).

2. Procédé suivant la revendication 1, caractérisé en ce qu'on prépare la 9-β-D-arabinofurannosyl-2-amino-6-méthoxy-9H-purine.

3. Procédé suivant la revendication 2, caractérisé en ce que l'ester pharmaceutiquement acceptable est choisi parmi les substances qui suivent :
9-(5-O-(4-Méthylbenzoyl)-β-D-arabinofurannosyl)-6-méthoxy-9H-purine;
9-(5-O-(4-Aminobenzoyl)-β-D-arabinofurannosyl)-6-méthoxy-9H-purine et
6-Méthoxy-9-(2-O-pentanoyl-β-D-arabinofurannosyl)-9H-purine.

4. Méthode de préparation d'un médicament, caractérisée en ce que
a) on prépare la 9-β-D-arabinofurannosyl-2-amino-6-méthoxy-9H-purine ou un sel, éther, ester, ou sel d'un tel ester, par un procédé suivant l'une quelconque des revendications 1 à 3 et
b) on associe le produit de l'étape a) à un ou plusieurs excipients ou véhicules pharmaceutiquement acceptables et, éventuellement, un ou plusieurs autres agents physiologiquement actifs.

5. Procédé suivant la revendication 4, caractérisé en ce qu'il comprend l'étape supplémentaire de transformation du mélange de l'étape b) en un comprimé ou une gélule.
